# EUROPÄISCHE PATENTSCHRIFT

(11) **EP 0 364 786 B1**
(45) Veröffentlichungstag und Bekanntmachung des Hinweises auf die Patenterteilung: **16.03.1994**
(21) Anmeldenummer: 89118043.2
(22) Anmeldetag: 29.09.1989
(51) Int. Cl.: G01N 33/36, D01B 3/02, B07C 5/34

(54) **Erkennung von Fremdgut in Textilfasern**
Identification of foreign matter in textile fibres
Identification de matière étrangère dans les fibres textiles

(30) Priorität: 11.10.1988 CH 3803/88
(43) Veröffentlichungstag der Anmeldung: 25.04.1990
(73) Patentinhaber: MASCHINENFABRIK RIETER AG, CH-8406 Winterthur (CH)
(72) Erfinder: Oehler, Oskar, Dr., CH-8032 Zürich (CH); Oehler, Reinhard, CH-8415 Berg am Irchel (CH); Demuth, Robert, CH-8309 Nuerensdorf (CH); Anderegg, Peter, CH-8400 Winterthur (CH)

(56) Entgegenhaltungen:
- EP-A- 0 000 033
- EP-A- 0 285 602
- DE-A- 3 708 188
- FR-A- 2 556 837
- GB-A- 2 095 828
- US-A- 3 750 461
- US-A- 4 095 475
- RÖMPPS CHEMIELEXIKON, 9. Aufl.; S. 3908-3909#

## Beschreibung

Die Erfindung betrifft eine Vorrichtung und ein Verfahren zur Erkennung von Fremdgut in einem Fasergutstrom der Textilindustrie insbesondere der Spinnerei gemäss Oberbegriff des ersten Vorrichtungs- und des ersten Verfahrensanspruches. Die Erfindung umfasst die Gebiete der Sensorik, der Akustik, der Optik und Elektronik sowie der Informationsverarbeitungstechnik. Es handelt sich dabei um ein Verfahren und eine Vorrichtung zur Erkennung von Schnur-, Gewebe-, Band-, oder folienförmigen Fremdgut in einem textilen Rohfasergutstrom.

Bei Textil-Rohfasern, wie Rohbaumwolle, ist es nicht zu vermeiden, dass Fremdgut ins Rohgut gelangt. Es können dies nebst Metallteilen auch Gewebestücke und Plastikfolien sein, sowie Bindematerial wie Schnüre, Bänder, Fäden etc.

Problematisch ist dabei die Abtrennung von Materialien in ihren Grundformen, die ähnliche Struktur und Beschaffenheit wie das Rohgut aufweisen, bspw. Schnurmaterial aus Jute oder Kunstfasern. Wird dieses letztere Fremdgut nicht rechtzeitig ausgeschieden, so durchläuft es den gesamten Reinigungs-, Kardierungs- und Faserparallelisierungsprozess, wird dabei in die faserförmigen Ausgangsmaterialien aufgelöst und schliesslich zusammen mit dem Nutzgut zu Fäden versponnen. Beim Spinnen oder auch erst beim Webvorgang treten infolge der niedrigen Zugfestigkeit oder schlechten Haftung zwischen Fremd- und Nutzfasern Fadenbrüche auf. Diese führen zu sehr unerwünschten Stillständen der Spinn- und der Webmaschinen. Zusätzliche Probleme treten beim Einfärben der mit Fremdfasern versetzten Fäden oder Geweben auf, da Nutz- und Fremdfasern verschiedene Färbeeigenschaften aufweisen können. Besonders gravierend ist dieses Problem, wenn es sich beim Fremdgut um Kunststoffe und beim Nutzgut um ein natürliches Produkt wie Baumwolle handelt.

Es ist daher ein grosses Interesse vorhanden, diese schwer erkennbaren Fremdstoffe nachzuweisen und auszuscheiden, bevor sie vom Kardierprozess erfasst werden.

Beispielsweise ergeben Schnüre oder Gewebe, die zusammen mit Baumwoll-Flocken eine Lichtschranke durchlaufen, durchaus spezifische Signale ab. Die Lichtintensitäts-Aenderung beim Passieren der Nutzgut-Flocken, resp. des Fremdguts kommen durch Lichtstreuung zustande. Da die Faserdimensionen mit 1 - 50 mm von ähnlicher Grössenordnung sind wie die Lichtwellenlänge, ist der Lichtstreu-Effekt sehr gross. Damit ergeben sich bei Verwendung von Licht zur Strukturdetektion sowohl für das Nutz- als auch für das Fremdgut sehr scharf strukturierte, jedoch äusserst komplizierte Signalkomponenten, die aber schwer voneinander unterscheidbar sind.

Aus EP-A-0285602 ist ein Verfahren und eine Vorrichtung zum Erkennen und Entfernen von Fremdstoffen aus Rohbaumwolle bekannt, welches die Rohbaumwolle mit Röntgenstrahlen durchstrahlt. Die Beschleunigungsspannung der verwendeten Röntgenröhre wird auf 8 bis 12 kV eingestellt, und die Durchstrahlung erfolgt an einer Stelle, an der die Rohbaumwolle auf ein Flockengewicht von etwa 10⁻¹ bis 10⁻³ g/Flocke aufgelockert wurde. Mit dieser Vorrichtung sollen vor allem Fremdfasern bis in eine Grössenordnung von 18 mg entfernt werden. Die mit einer solchen Röntgenröhre erzeugte Strahlung liegt unterhalb 0,25 nm.

Es hat sich jedoch gezeigt, dass, wenn mit einer Strahlungsart gearbeitet wird, deren Wellenlängen nicht von der Grössenordnung der Faserdimensionen ist, sondern eine Grösse der typischen Dimensionen der nachzuweisen Fremdstoffe aufweist, dass dann bessere Resultate zu erwarten sind. Bei Schnüren und Gewebefäden liegen diese Dimensionen im Bereich 0,1 - 3,5 mm.

Die Erzeugung, Handhabung und Detektion von anderer (als Licht) elektromagnetischer Strahlung der erwähnten Wellenlänge ist ebenfalls möglich, jedoch eher schwierig und aufwendig. Dieser 0,1 - 3,5 mm - Bereich liegt im Zwischengebiet der Mikrowellen- und Infrarotstrahlung.

Eine Alternative zur elektromagnetischen Strahlung bietet die Verwendung von Ultraschallwellen. Die Erzeugung eines entsprechenden Feldes einer Wellenlänge von 0,1 - 3,5 mm ist wegen der niedrigen Schallgeschwindigkeit der Luft von ca. 340 m/s nicht problematisch. Allerding ist die Temperaturabhängigkeit der Schallgeschwindigkeit von ca. 0,6 m/s K zu berücksichtigen.

Ultraschallsignale der gewünschten Frequenz werden in der Regel mit piezoelektrischen Keramikscheiben oder piezofolienförmigen Elementen erzeugt. Wegen deren grosser akustischen Härte (h = E p, wo E: Elastizitätsmodul, p: Dichte) gegenüber derjenigen der Luft, ist die Anpassung und damit die Energieübertragung vom Sensorelement an die Luft zu berücksichtigen. In Analogie zur Methode der optischen Vergütung kann die Anpassung an sich durch Zwischenschichten geeigneter akustischer Härte verbessert werden. Bei Verwendung einer Piezokeramik als Schallgenerator werden Lambdaviertel-Platten aus Kunststoff zur Schallimpedanzanpassung verwendet.

Es ist nun Aufgabe der Erfindung ein Verfahren und eine Vorrichtung zu schaffen, die es erlauben, faser-, band- oder gewebeförmige Fremdstoffe in einem Nutzfaserstrom zu erkennen und auszuscheiden.

Die Aufgabe wird erfindungsgemäss durch die im Kennzeichen des ersten Verfahrens - und ersten Vorrichtungsanspruches definierte Erfindung gelöst.

Die Erfindung wird nun anhand von lediglich Ausführungswege darstellenden Zeichnungen näher erläutert.

Es zeigt:
- Fig. 1: ein erfindungsgemässes Verfahren resp. eine erfindungsgemässe Vorrichtung, schematisch dargestellt,
- Fig. 2a, b, c: je einen zeitlichen Verlauf eines Detektorsignales, erhalten aufgrund eines Nutz- und/oder Fremdgutes in einem in Fig. 1 dargestellten Fasergutstrom,
- Fig. 3: eine Variante des erfindungsgemässen Verfahrens resp. der erfingungsgemässen Vorrichtung,
- Fig. 4a, b, c: je einen zeitlichen Verlauf eines Detektorsignales, erzeugt aufgrund eines reinen resp. eines mit Fremdgut durchsetzten Fasergutstromes,
- Fig. 5: eine weitere Variante der erfindungsgemässen Vorrichtung, wobei das Transmissionssystem durch ein Reflexionssystem ersetzt worden ist.
- Fig. 6: eine Ausführungsvariante des erfindungsgemässen Systemes, schematisch dargestellt,
- Fig. 7a, b: die Struktur eines Raumfrequenzspektrums bei einem mit Fremdgut durchsetzten Nutzfaser-Flockenstrom,
- Fig. 8: eine Variante des Verfahrens und der Vorrichtung von Fig. 3 und 5,
- Fig. 8a, b, c: je eine Struktur eines Raumfrequenzspektrums der Signale von der Vorrichtung von Fig. 8.
- Fig. 9 - 11: je eine Ausscheidevorrichtung zur Durchführung des erfindungsgemässen Verfahrens.

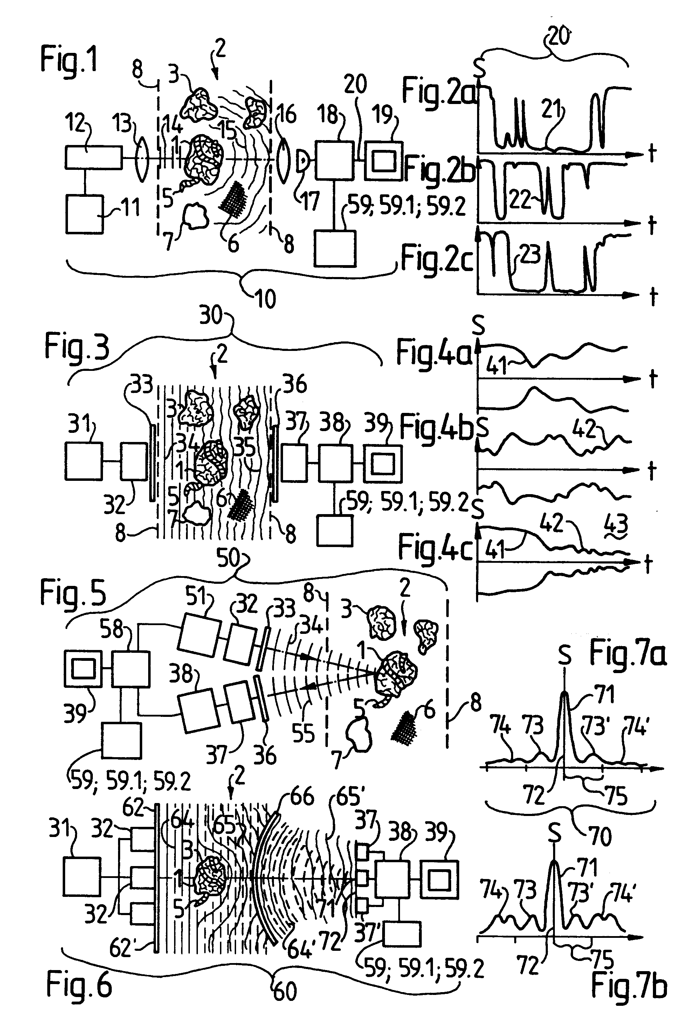

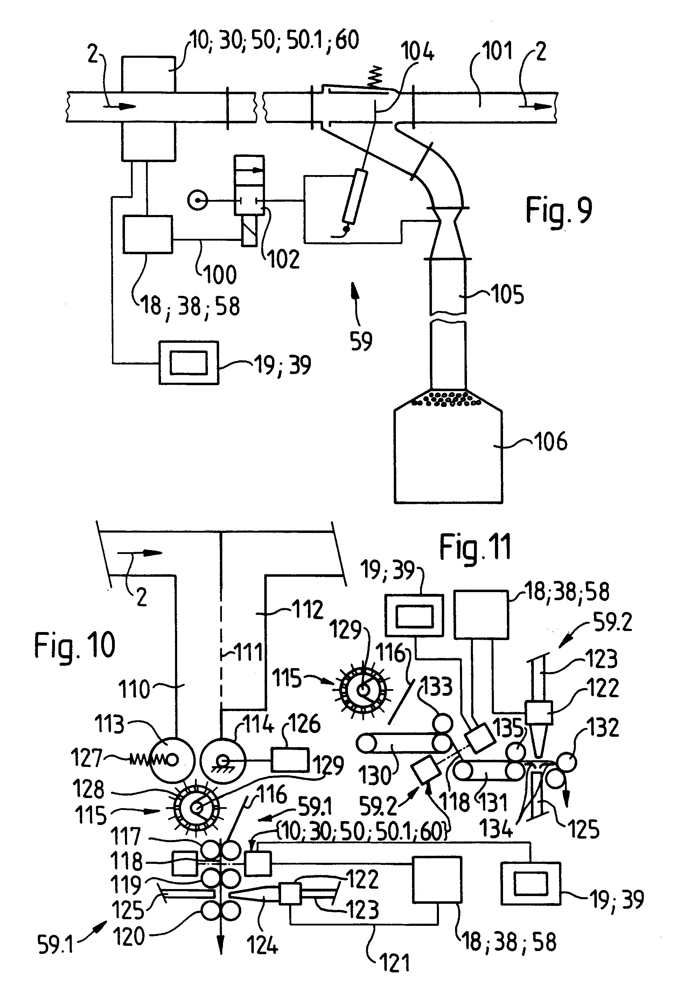

Fig. 1 zeigt die Darstellung einer optischen Vorrichtung zur Erfassung der Lichtransmission eines Nutzgut 3 und Fremdgut 5 resp. 6 resp. 7 enthaltenden Messgutstromes 2. Beim Nutzgut 3 handelt es sich entweder um flockenförmiges Fasermaterial 3, wie Rohbaumwoll-Flocken in einem Transportrohr 8, oder um ein Nutzfaservlies (Fig. 10 + 11) in einem Vliesstapel. Das Fremdgut kann beispielsweise aus Schnüren 5, Geweben 6 oder Folienstücken 7 bestehen. Der Lichtstrahl 14, welcher in der, aus Speisung 11 und Lichtemitter 12 bestehenden Quelle erzeugt und gegebenenfalls mittels der Optik 13 auf das Messgut 1 im Messgutstrom 2 gerichtet wird, erfährt durch das Messgut 1 eine Strahlveränderung 15. Diese Veränderung ist vor allem durch Streuung an den Fasern und gegebenenfalls durch Lichtabsorption in gefärbtem Messgut bedingt. Beim Durchlaufen des Messgutstromes 2 (auch Fasergutstrom genannt) wird daher am Lichtdetektor 17, der hinter einer gegebenenfalls vorhandenen Detektoroptik 16 angeordnet ist, ein sich zeitlich variierendes Lichtintensitäts-Signal empfangen und in ein elektrisches Signal umgesetzt. Letzteres wird in der Signalaufbereitungsvorrichtung 18 verarbeitet, bspw. verstärkt, geglättet oder einer Signalhöhen-Diskriminierung unterzogen. Zusätzlich kann in der Signalaufbereitungsvorrichtung eine Strukturanalysenvorrichtung zur Detektion des Messgutes 1 vorhanden sein, welche gegebenenfalls das Messgut bezüglich vorhandenem Fremdgut 5 resp. 6 resp. 7 beurteilt. Die Displayvorrichtung 19, die mit der Signalaufbereitungsvorrichtung 18 verbunden sein kann, vermittelt eine visuelle Darstellung der empfangenen Struktur. Diese Vorrichtung dient zur Vornahme von Kontrollmessungen. Es kann sich bei 19 bspw. um einen Schreiber oder um ein Kathodenstrahl-Oszilloskop handeln. Zur Abtastung des Messgutstromes 2 können entweder mehrere Lichtstrahlen nebeneinander angeordnet sein, oder es kann bspw. der Messgutstrom 2 mittels einer drehbaren Optik 13 beim Lichtemitter 12 und einer entsprechenden Optik 16 beim Detektor 17 abgetastet werden.

Die Fig. 2a, 2b und 2c zeigen den zeitlichen Verlauf des Lichtsignales am Detektor 17 beim Passieren verschiedener Messgutproben 1 durch den Lichtstrahl 14 eines Lasers. In diesem Falle erübrigte sich die Verwendung einer zusätzlichen Optik 13 resp. 16. Es ist zu beachten, dass eine sehr lose Baumwollflocke 3 einer Dichte von 0,03 g/cm³ bereits zu einer sehr grossen Abschwächung 21 des Lasersstrahlers 14 führt, wie aus Fig. 2a hervorgeht. Das hängt damit zusammen, dass die einzelnen Baumwollfasern wegen des, mit der Lichtwellenlänge vergleichbaren, Durchmessers zu einer starken Lichtstreuung Anlass geben. Fig. 2b zeigt das Signal am Lichtdetektor 17 für das Passieren eines Schnurknäuels 5. Die einzelnen Schnurstückdurchgänge können aufgrund des Licht-Transmissionsabfalles eindeutig detektiert werden. Fig. 2c veranschaulicht die Situation des Durchlaufes einer, mit Schnur 5 durchmischten Baumwollflocke. Es wurde die für Fig. 2b verwendete Schnur verwendet.

Fig. 3 zeigt eine Messvorrichtung 30, bei der als Probestrahl ein Ultraschallfeld 34 verwendet wird. Diese Vorrichtung besteht aus einem Signalgenerator 31, bspw. einem Sinus- oder Rechteck-Generator, dessen Ausgangssignal einem als Ultraschall-Sender 32 betriebenen Ultraschall-Wandler zugeführt wird. Das emittierte, gegebenenfalls durch eine Ultraschalloptik 33 modifizierte, Schallfeld 34 trifft auf das Messgut 1 und wird dort gestreut oder absorbiert. Das gestörte Unterschallfeld 35 wird gegebenenfalls nach einer Beeinflussung durch die Ultraschalloptik 36 vom Ultraschallempfänger 37 detektiert und das entsprechende elektrische Signal der Signalaufbereitungsvorrichtung 38 zugeführt. Dort wird die Enveloppe des hochfrequenten Signales bestimmt und anschliessend zu Kontrollzwecken, der Displayvorrichtung 39 zugeführt. Zur Erkennung des Fremdgutes 5 resp. 6 resp. 7 können in der Signalaufbereitungsvorrichtung 38 Bildstrukturerkennungsmittel vorhanden sein. Ebenso wird in der Signalaufbereitungsvorrichtung 38, beim vorliegenden erkannten Fremdgutes gegebenenfalls ein Signal erzeugt, das zur Umlenkung des Messgutstromes 2 dienen kann.

In Fig. 4a, 4b und 4c sind typische zeitliche Veränderungen der Enveloppen des Unterschallsignales für die Wechselwirkung des Messgutes 1 mit einem Ultraschallfeld 34 dargestellt. Fig. 4a zeigt den Durchlauf einer reinen Baumwollflocke, Fig. 4b denjenigen eines Schnurknäuels und Fig. 4c entspricht dem Passieren einer mit der Schnur vermischten Baumwollflocke. Aufgrund der Frequenz des Ultraschallsignales von 98 kHz, entspricht die Wellenlänge mit 3,5 mm dem Schnurdurchmesser, übersteigt jedoch den Durchmesser der Baumwollfasern um ca. einen Faktor 100. Aus diesem Grunde zeigt sich in Fig. 4a eine weich strukturierte Ultraschalldämpfung 41, während die Schnur 5 nach Fig. 4b eine scharfe Strukturierung 42 der Ultraschallsignalenveloppe aufweist. In der Enveloppe der Ultraschallintensität 43 für das Passieren der mit der Schnur vermischten Baumwollflocke, ist die weiche Struktur 41 der Flocke 3 von der Struktur 42 der Schnur 5 deutlich unterscheidbar.

Die Fig. 5 zeigt eine weitere Methode zur Messung der Wechselwirkung eines Ultraschallfeldes mit dem Messgut 1. Es tritt wiederum ein Ultraschallsignal 34 mit dem Messgut 1 in Kontakt, welches in Flockenform ein Transportrohr durchquert, oder ein Vliess (nicht gekennzeichnet) 4 bildet. Im Gegensatz zur Vorrichtung gemäss Fig. 3 produziert der Ultraschallsender 32 mittels der elektrischen Signale des Ultraschall-Pulsgenerators 51, kurze Ultraschall-Wellenzüge, anstelle von Signalen von zeitlich konstanter Amplitude. Dieselben werden am Messgut 1 reflektiert. Die Echo-Signale 55 werden entweder vom Ultraschall-Sender 32 selbst empfangen oder von einem zweiten Ultraschallempfänger 37 detektiert und in der Signalaufbereitungsvorrichtung 38 bearbeitet. Aus der Zeitverzögerung zwischen dem vom Sender 32 emittierten Ultraschallpuls und dem vom Empfänger 32 resp. 37 empfangenen Echo, lässt sich im Korrelator 58 die Distanz des schallreflektierenden Messgutes 1 von den Ultraschallwandlern 32 resp. 37 ermitteln. Gegebenenfalls können im Korrelator enthaltene Bildstrukturerkennungsmittel das Fremdgut 5 resp. 6 resp. 7 detektieren und entsprechende Signale zur Ablenkung des Messgutstromes 2 produzieren.

Wie bereits gezeigt (Fig. 4a, 4b und 4c), erlaubt die erfindungsgemässe Ultraschallmethode eine Unterscheidung der losen Struktur einer Baumwollflocke 3, von derjenigen einer fest geflochtenen Schnur 5. Es stellt sich nun die Frage der Auswertung dieser Information. An sich sind die Methoden der Struktur-Erkennung (pattern recognition) dazu geeignet, diese Aufgabe zu lösen. Mit den heute zur Verfügung stehenden schnellen Rechnern ist es durchaus möglich, das Vorhandensein von akustisch dichtem Fremdgut, wie Schnüre 5 und Gewebe 6 im losen Flockenstrom 2 oder in einem Vlies (nicht gezeigt) zu erkennen. Da aber der Messgutstrom 2 ständig auch seitlich überwacht werden muss, ist entweder eine grosse Anzahl nebeneinander angeordneten Messvorrichtungen 32 resp. 37 notwendig, oder ein Probenstrahl muss seitlich über den Messgutstrom 2 resp. das Messgutvlies 118 (Fig. 10 + 11)) geführt werden (scanning).

Es stellt sich nun die Frage nach einer messtechnisch einfachen Erfassung der Fremdgutstruktur. In diesem Zusammenhang kann die Raumfrequenzanalyse des, durch das Messgut gestörte, Ultraschall-Signales zu einer wesentliches Datenreduktion führen. Eine entsprechende Apparatur wird in der im folgenden beschriebenen Figur gezeigt.

Fig. 6 zeigt den Aufbau einer Vorrichtung 60 zur Durchführung einer Ultraschall-Raumfrequenzanalyse. Diese Apparatur 60 besteht aus dem Signalgenerator 31, der Ultraschall-Sendevorrichtung 32, welche ein ausgedehntes, ebenes oder gekrümmtes Ultraschallfeld 64 erzeugt und der Ultraschall-Sammeloptik 66 sowie dem Ultraschall-Detektor 37. Die Ultraschall-Sendevorrichtung 62 wird entweder durch eine Anordnung verschiedener Einzelsender 32, 32′, .... oder durch einen einzelnen, oder gegebenenfalls wenig grossflächigen Ultraschallsender 62′ von geeigneter Form gebildet. Im Ultraschallfeld 64 ist eine Ultraschall-Sammeloptik 66 angeordnet, die in Abwesenheit des Messgutes 1 das Feld 64 auf einen Punkt, den Fokalpunkt 71, fokussiert. Auf die Ultraschall-Sammeloptik 66 kann verzichtet werden, falls die ausgedehnte Ultraschall-Sendevorrichtung 62 eine geeignete fokussierende Krümmung aufweist. Vorzugsweise wird aber, wie in Fig. 6 abgebildet, von einem ebenen Schallfeld 64 ausgegangen, das durch eine, hinter dem Messgutstrom 2 angeordnete, Sammeloptik 66 gebündelt wird.

Falls sich ein Messgut 1 im Schallfeld befindet, wird das Feld gestört. Demzufolge ist eine Bündelung des Feldes 65′ auf den Fokalpunkt 71 nicht mehr möglich: Betrachtet wird die Ultraschall-Intensitätsverteilung 70 in der Fokalebene 72, d.h. in der, den Fokalpunkt 71 enthaltenden Parallelebene zum ebenen Ultraschallfeld 64. Diese Intensitätsverteilung 70 gibt spezifische Auskunft über die Ultraschall-Streueigenschaften des Messgutes. In Analogie zur Optik stellt sie die Fourier-Transformierte des streuenden Objektes 1, das einem ebenen Feld 64 ausgesetzt ist, dar. Die Intensitätsverteilung 70 in der Fokalebene 72 wird daher als Raumfrequenz-Spektrum 70 bezeichnet.

Das Ultraschall-Raumfrequenz-Spektrum 70 wird anhand von Fig. 7a und 7b näher erläutert. Der zentrale Fokalpunkt 71, auf welchem ohne Störung durch das Messgut 1 das Ultraschallfeld 64′ fokussiert ist, entspricht der Raumfrequenz 0. Ein schallstreuendes Objekt 1 im ebenen Feld 64 führt zu einer strukturierten, mit den Seitenbändern 73, 73′, 74, 74′ versehenen Raumfrequenzspektrum 70. Dabei entsprechen die dem Fokalpunkt 71 benachbarten Bändern 73, 73′ tiefen Raumfrequenzen und die entfernteren Bändern 74, 74′ hohen Raumfrequenzen. Ebenso, wie in der Optik die niedrigen Raumfrequenzen die Grobstruktur beschreiben und die hohen Raumfrequenzen für die Schärfe des Bildes verantwortlich sind, entsprechen die niedrigen Raumfrequenzen 73, 73′ den weich strukturierten Ultraschallsignalen, wie sie bspw. nach Fig. 4a einer reinen Baumwollflocke zugeordnet werden können. Das entsprechende Raumfrequenzspektrum 70 ist in Fig. 7a dargestellt. Bemerkenswert ist die niedrige Signalgrösse bei hohen Raumfrequenzen 74 resp. 74′.

Ist hingegen das Ultraschallsignal scharf strukturiert, wie das bspw. nach Fig. 4b für eine Schnur im Ultraschallfeld gefunden wurde, so weist das entsprechende Raumfrequenzspektrum 70 bei hohen Raumfrequenzen 74 resp. 74′ beachtliche Signalgrössen auf. Da selbst in der Durchmischung einer Baumwollflocke 2 mit einer Schnur 5 die zwei Ultraschall-Strukturtypen, wie Fig. 4c zeigt, unterschieden werden können, ist eine zuverlässige Erkennung des Fremdgutes 5 resp. 6 resp. 7 aufgrund einer Raumfrequenzanalyse möglich. Das Problem der Strukturerkennung vereinfacht sich damit wesentlich. Die Raumfrequenzanalyse bedeutet damit eine enorme Reduktion der Datenmenge.

Fig. 7b zeigt das entsprechende Raumfrequenzspektrum 70 mit dem, gegenüber des Spektrums der reinen Baumwollflocke (7a), erhöhten Signal bei hohen Raumfrequenzen 74 resp. 74′.

Bei der Vorrichtung, wie sie mit Fig. 6 beschrieben ist, wurde davon ausgegangen, dass sich das Messgut 1 in einem ebenen Ultraschallfeld 64 befindet, das nachträglich mit Hilfe der Ultraschall-Sammeloptik 66 fokussiert wird. Prinzipiell ist es, wie bereits erwähnt, auch möglich eine fokussierende Senderanordnung zu verwenden und das Messgut 1 im divergenten Ultraschallfeld zu positionieren. Es kommt in diesem Falle auch ein Raumfrequenzspektrum 70 zustande. Der Massstabsfaktor 75 des Raumfrequenzspektrums ist nun allerdings von der Lage des Messgutes 1 in diesem divergenten Ultraschallfeld abhängig. So besitzen Messgutteile, die sich unmittelbar hinter der Sammeloptik 62 resp. 66 befinden, einen grösseren Raumfrequenz-Massstabsfaktor 75 als Messgutteile, die in der Nähe des Fokalpunktes 71 liegen. Somit ist es durchaus möglich, dass die Rauhigkeit eines Objektes 1, je nach Lage in divergenten Ultraschallfeld, grossen Raumfrequenzwerten 74 resp. 74′ oder kleinen Raumfrequenzwerten 73 resp. 73′ zugeordnet ist. Dieses Problem wird im schwach konvergenten Ultraschallfeld entschärft. Allerdings wären in diesem Falle grosse Vorrichtungs-Abmessungen notwendig, was wegen der hohen Luft-Absorptionsverluste des erforderlichen hochfrequenten Ultraschalles einer Wellenlänge im Bereiche von 1 bis 3 mm ungünstig wäre.

Es ist festzuhalten, dass im ebenen Ultraschallfeld, in Analogie zum ebenen optischen Feld, der Massstabsfaktor 75 unabhängig von der Lage des Messgutes im Ultraschallfeld 64 ist. Objekte 1 die sich in der Nähe des Senders und solche, die sich direkt vor der Fokussieroptik 66 befinden, geben somit zu demselben Raumfrequenz-Spektrum 70 Anlass, was eine für die Auswertbarkeit des Raumfrequenz-Spektrums von räumlich ausgedehnten Objekten wichtige Feststellung ist.

Es ist nicht unbedingt notwendig, dass das Ultraschallfeld 64, das mit dem Messgut 1 in Wechselwirkung steht, eben, d.h. in zwei Dimensionen konstant ist. Falls das Messgut 1 beispielsweise als Messgutstrom 2 bewegbar ist, oder gegebenenfalls die Ultraschall-Sendevorrichtung 62 über das Messgut 1 geführt werden kann, ist es durchaus sinnvoll, dass lediglich verlangt wird, dass das Ultraschallfeld 64 quer zum Messgutstrom 2, resp. quer zur Bewegungsrichtung der Ultraschall-Sendevorrichtung 62 konstant ist. Das Feld kann in diesem Falle in Abwesenheit des Messgutes nicht auf einen Fokalpunkt 71, sondern lediglich auf eine Linie, die Fokallinie 71′, fokussiert werden. Eine Raumfrequenzanalyse kann durch Abtasten des Ultraschallfeldes 65′ längs der Normalen zur Fokallinie 71′ in der Fokalebene 72 durchgeführt werden.

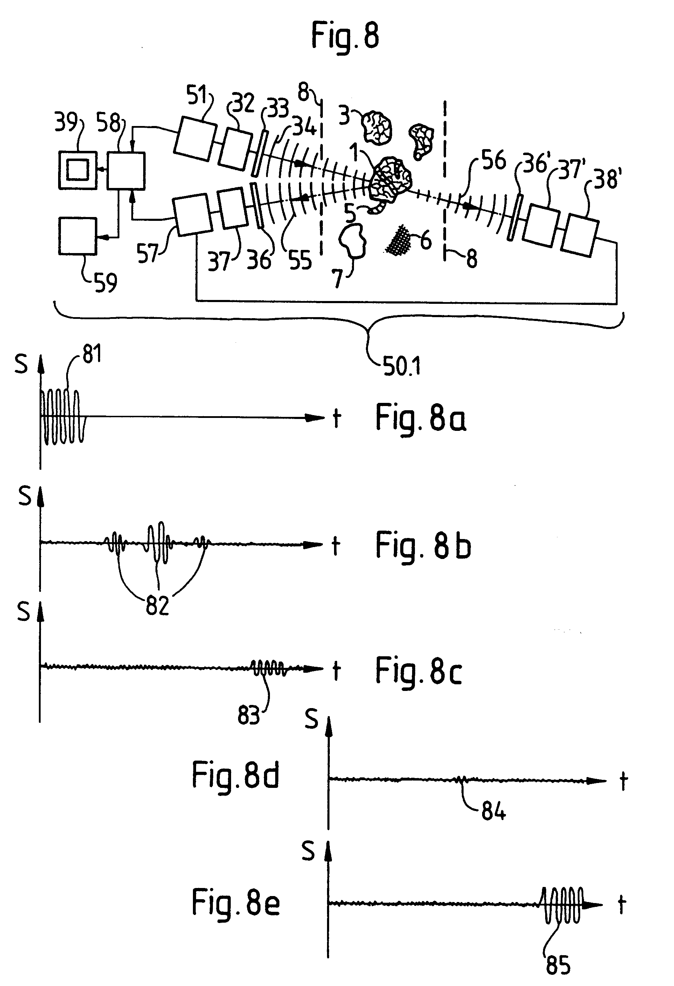

Als Ergänzung zu der in Fig. 5 beschriebenen Methode verwendet die Methode nach Fig. 8 eine zusätzliche Ultraschallempfängereinheit 36′, 37′, 38′, die den durch das Messgut transmittierten Ultraschallpuls 56 detektiert. Der zeitliche Verlauf ist je in Fig. 8a - 8e dargestellt. Enthält das Messgut 2 kein Fremdgut 5 resp. 6 resp. 7, so bildet sich kein ausgeprägter Ultraschall-Echopuls 84 aus und der ausgesendete Ultraschall-Puls 81 gelangt in weniger geschwächter Form 85 zur Ultraschall-Empfängereinheit 36', 37', 38'. Führt das Messgut 2 hingegen Fremdgut 5 resp. 6 resp. 7 mit sich, so bilden sich von der Struktur, Grösse und Lage abhängige Ultraschall-Echopulse 82 aus, die vom Ultraschallsender-Vorrichtung 32, 33 selber oder von einer Ultraschallempfangs-Vorrichtung 36, 37 empfangen werden. Das Fremdgut 5 resp. 6 resp. 7 kann auch eine Abschwächung des transmittierten Ultraschallpulses 83 bewirken, der von der Ultraschallempfängereinheit 36', 37', 38' empfangen wird. Die beiden Signale 82 und 83 - bzw. im Falle, dass das Messgut 2 kein Fremdgut 5 resp. 6 resp. 7 enthält, die Signale 84 und 85 - werden in der folgenden Signalverarbeitungs-Vorrichtung 57 aufbereitet, dem Korrelator 58 zugeleitet, der seinerseits im Falle der Detektions von Fremdgut 5 resp. 6 resp. 7 die Ausscheidevorrichtung 59 aktiviert.

Die Signalverarbeitungs-Vorrichtung regelt in Abhängigkeit der Signalstärke der transmittierten Ultraschall-Pulser (56) die Verstärkung der Echopulse 55, was bewirkt, dass die relativ schwachen Echopulse 55 von Fremdgut 5/6/7 sicher ausgewertet werden können ohne dass bei einem reinen Fasergutstrom einzelne Baumwoll-Flocken, also nicht Fremdgut, erfasst werden.

Die Kennzeichnung der Ultraschallempfängereinheit mit den Nummern 36', 37' und 38' deutet darauf hin, dass es sich um dieselben Elemente handelt, die in Figur 3 und 6 beschrieben, lediglich in dieser Variante verwendet.

Ebenso sei erwähnt, dass die Signalaufbereitungsvorrichtung 38 oder Korrelator 58 nicht nur eine Displayvorrichtung 39 steuert, sondern hauptsächlich eine Ausscheidevorrichtung. Eine solche Ausscheidevorrichtung ist in der europäischen Patentschrift mit der Veröffenlichungsnummer 0 000 033 vom Anmelder beschrieben. Die Steuerung würde hauptsächlich mit den eigentlichen Ausscheideelementen, nämlich einem Steuerventil und einer in einer pneumatischen Förderleitung eingebauten Rohrweiche bestehen, welche aufgrund eines Steuersignales umschaltet und dem mit Verunreinigung betreffenden Anteil in einen Behälter abzweigt.

Die Fig. 9 zeigt eine Ausscheidevorrichtung 59, welche von der Signalaufbereitungsvorrichtung 18 resp. 38 resp. Korrelator 58 über die Steuerleitung 100 gesteuert wird. Die Signalaufbereitungsvorrichtung erhält ihrerseits die Signale der Messvorrichtung 10 resp. 30 resp. 50 resp. 50.1 resp. 60.

Die Ausscheidevorrichtung 59 ist an sich aus der bereits erwähnten europäischen Patentanmeldung 0 000 033 bekannt, weshalb hier nur noch die wesentlichen Elemente nochmals erwähnt werden.

Der Fasergutstrom 2 wird in einem pneumatischen Förderrohr 101 einem hier nicht weiter genannten Ziel in der Putzerei der Spinnerei zugeführt.

Stellt die vorgenannte Messvorrichtung ein Fremdgut fest, errechnet die Signalaufbereitungsvorrichtung 18 resp. 38 resp. 58 den Zeitpunkt, zu welchem ein Ventil 102 einen Pneumatikzylinder 103 derart betätigt, dass eine Rohrweiche 104 den Fasergutstrom 2 in einen Bypass 105 leitet. Dadurch wird das ausgeschiedene Fremdgut in einen Ausscheidebehälter 106 gefördert.

Zur Information sei hingewiesen, dass die Nummerierung der Elemente der Abscheidevorrichtung nicht mit der Nummerierung in der vorerwähnten europäischen Patentanmeldung übereinstimmt.

Im weiteren sind diejenigen Elemente, welche bereits früher erwähnt wurden, hier nicht mehr erwähnt, sondern lediglich auf der Fig. 9 mit den bekannten Kennzeichnungsnummern versehen.

Die Fig. 10 zeigt eine Variante einer Ausscheidevorrichtung 59.1.

Darin wird der Fasergutstrom 2 in einen Abscheider 110 geführt, in welchem sich das Fasergut bis zu einem vorgegebenen Niveau anhäuft und die Luft durch ein Lochblech 111 in einen Luftabführschacht 112 entweichen kann und dort durch eine Unterdruckquelle abgesaugt wird.

Das im Abscheider 110 angehäufte Fasergut 2 wird durch Speisewalzen 113 und 114 einer Auflösewalze 115 zugespeist, welche das Fasergut kontinuierlich in feine Flocken auflöst und gegen ein Prallblech 116 schleudert.

Von diesem Prallblech 116 gelangen die Fasern zwischen ein erstes Klemmwalzenpaar 117, welche die Fasern oder feinen Faserflocken zu einem feinen Faservlies verdichten, welches in folge der Wirrlage der Fasern eine genügende Festigkeit aufweist. Dieses Faservlies 118 gelangt zu einem zweiten Klemmwalzenpaar 119 und anschliessend zu einem dritten Klemmwalzenpaar 120. Zwischen dem ersten und zweiten Klemmwalzenpaar wird das Faservlies 118 durch die Messvorrichtung 10 resp. 30 resp. 50 resp. 50.1 resp. 60 gemessen und das Signal per Signalaufbereitungsvorrichtung 18 resp. 38 resp. 58 zugeleitet.

Die Signalaufbereitungsvorrichtung 18 resp. 38 resp. 58 steuert über Steuerleitungen 121 eine vorgegebene Anzahl pneumatischer Ventile 122, welche eingangseitig mit einer Druckluftleitung 123 und ausgangsseitig mit einer Düse 124 versehen sind. Die Düsen 124 ragen zwischen das zweite Klemmwalzenpaar 119 und das dritte Klemmwalzenpaar 120 und blasen Druckluft auf Kommando der zuvor erwähnten Signalaufbereitungsvorrichtung in das Faservlies 118 und zwar derart, dass entsprechend der Anzahl Düsen 124 verteilt auf die ganze Breite des Faservlieses 118 nur derjenige Teil des Faservlies in eine Aspirationsleitung 125 geblasen wird, in welchem sich das entsprechende, von der Messvorrichtung ermittelte Fremdgut befindet. Es versteht sich, dass möglichst viele oder verschiebbare Düsen 124 und damit die entsprechende Anzahl Ventile 122 auf der Vliessbreite verteilt werden, um möglichst kleine, zu entfernende Vliesstücke zu erhalten.

Im weiteren versteht es sich ebenfalls, dass die Signalaufbereitungsvorrichtung 18 resp. 38 resp. 58 die Zeit errechnet, zwischen dem Feststellen des Fremdgutes und dem Zeitpunkt des Ausblasens. Das vom letzten d.h. dritten Klemmwalzenpaar 120 abgegebene Vliess kann z.B. in einen Speiseschacht einer Karde geführt werden. Gegebenenfalls kann dieses Vlies neuerdings durch eine Auflösewalze in Flocken aufgelöst werden.

Im weiteren sei erwähnt, dass die Speisewalze 114 durch einen entsprechend gesteuerten Motor 126 angetrieben und dass die Speisewalze 113 bewegbar gelagert und mittels Federn 127 gegen die angetriebene Speisewalze 114 gepresst wird, um die ausgetragenen Faserflocken resp. das Fasergut aus dem Abscheider 110 zu einer Watte zu verdichten, welche anschliessend durch die Auflösewalze 115 in feine Flocken aufgelöst wird.

Im weiteren kann die Auflösewalze 115 hohl und mit Durchgangslöchern 128 versehen werden, damit hilfweise über eine Luftdüse 129 an derjenigen Stelle der Rohwalzen Luft durch die Bohrungen 128 geblasen werden kann, dass die feinen Faserflocken von der Auflösewalze 115 losgelöst und gegen das Prallblech 116 gefördert werden können.

Die Fig. 11 zeigt insofern eine Variante der Vorrichtung von Fig. 10, als das Faservlies 118 nicht in vertikaler Richtung durch drei Walzenpaare gefördert wird, sondern durch zwei Förderbänder und ein Klemmwalzenpaar, wobei das erste Förderband mit 130, das zweite mit 131 und das Klemmwalzenpaar mit 132 gekennzeichnet ist.

Dabei sind die aus der Fig. 10 bekannten Elemente mit denselben Bezugszeichen versehen und nicht nochmals erklärt. In Bezug auf die Bezeichnung der Detektions und Ausscheide-Elemente sei auf Fig. 10 hingewiesen.

Die von der Auflösewalze aufgelöste und vom Prallblech 116 geführten feinen Flocken fallen auf das Förderband 130 und werden ausgangsseitig mittels einer Presswalze 133 zu einem dünnen Faservlies verdichtet, welches auf das zweite Förderband 131 gegeben wird. Zwischen dem ersten und zweiten Förderband wird das Faservlies 118 von der Messvorrichtung 10 resp. 30 resp. 50 resp. 50.1 resp. 60 gemessen. Das Ausgangssignal dieser Messvorrichtung genannt wie bisher beschrieben zur Signalaufbereitungsvorrichtung 18 resp. 38 resp. 58, welche ebenfalls den Zeitpunkt errechnet, zu welchem eine der Düsen 124 Luft durch das Faservlies 118 bläst, welches sich zu diesem Zeitpunkt zwischen dem zweiten Förderband 131 und dem anschliessend vorgesehenen Quetschwalzenpaar 132 befindet.

Zur Führung des Vlieses im Bereich der Düsen 124 sind zwei Leitbleche 134 vorgesehen, welche eine Durchblasöffnung (nicht gekennzeichnet) für die Luft der Düsen 124 freilässt, so dass diese Luft zusammen mit dem weggetretenen Vliesteil von der Absaugleitung 125 aufgenommen werden kann.

Letztlich sei noch auf die Klemmwalze 135 hingewiesen, welche das Faservlies 116 ausgangsseitig des zweiten Förderbandes 131 gegen dieses klemmt, um für das Ausblasen der Fremdgutteile ein gutgeführtes Faservlies zu erhalten.

Bei den Varianten gemäss den Figuren 10 und 11 werden vorteilhafterweise die Methoden gemäss den Figuren 5 und 8 verwendet, da optimale Verhältnisse für die Detektion vorhanden sind. Die Fremguterkennungs-Vorrichtung werden hier als feste oder verschiebbare Tastköpfe (Scanning-Prinzip) ausgebildet.

Die am Beispiel der Ultraschall-Raumfrequenzanalyse aufgeführten Ueberlegungen gelten natürlich auch für eine entsprechende optische Raumfrequenzanalyse.

## Patentansprüche

1. Verfahren zur Erkennung von Fremdgut in einem Fasergutstrom (auch Messgutstrom genannt) in der Textilindustrie, in Form eines Luft-Faser-Gemisches oder in Form eines Vlieses, wobei das erstere in einem pneumatischen Transportrohr und das zweite frei oder mechanisch bewegbar transportiert wird,
dadurch gekennzeichnet,
- dass der Fasergutstrom mit einem vorgegebenen Wellenfeld in Wechselwirkung steht, dessen Wellenlängen in der Grössenordnung der typischen Dimensionen des nachzuweisenden Fremdguts liegen und das durch einen entsprechenden Wellenerzeuger gebildet wird, welcher mit einem Signalgenerator in Verbindung steht, und
- dass das durch die Wechselwirkung zwischen Messgut und Wellenfeld aufgrund von Wellenstreuung veränderte Wellenfeld von Wellenempfängern empfangen wird, und
- dass die Information über die Struktur des nachzuweisenden Fremdgutes den elektrischen Signalen von den Wellenempfängern entnommen wird.

2. Verfahren nach Anspruch 1, dadurch gekennzeichnet, dass das Wellenfeld des Wellenerzeugers durch eine Wellenoptik gebündelt wird.

3. Verfahren nach Anspruch 1, dadurch gekennzeichnet, dass das Wellenfeld durch Wellenstreuung und/oder Absorption verändert wird.

4. Verfahren nach Anspruch 1, dadurch gekennzeichnet, dass die Signale aufbereitet werden.

5. Verfahren nach Anspruch 1, dadurch gekennzeichnet, dass das durch den Fasergutstrom veränderte Wellenfeld nach einer Wellenbeeinflussung durch eine Wellenoptik von Wellenwandlern empfangen wird.

6. Verfahren nach Anspruch 1, dadurch gekennzeichnet, dass von mindestens einem Wellenwandler kontinuierlich ein Wellensignal ausgesandt und mittels mindestens einem weiteren Wellenwandler das durch das Messgut beeinflusste Wellensignal empfangen und, dass das durch das Messgut abgeschwächte oder gestreute Wellensignal gemessen wird.

7. Verfahren nach Anspruch 6, dadurch gekennzeichnet, dass ein im Vergleich mit den Querabmessungen des Messgutstromes ausgedehntes, ebenes Wellenfeld mit dem Messgut in Wechselwirkung steht.

8. Verfahren nach Anspruch 6, dadurch gekennzeichnet, dass ein im Vergleich mit den Querabmessungen des Messgutstromes ausgedehntes, lineares Wellenfeld mit dem Messgut in Wechselwirkung steht.

9. Verfahren nach Anspruch 6, dadurch gekennzeichnet, dass das Messgut mit einem konvergenten Wellenfeld, dessen Fokalpunkt sich ausserhalb des Messgutstromes befindet, in Wechselwirkung steht.

10. Verfahren nach Anspruch 7, dadurch gekennzeichnet, dass das Messgut mit einem ebenen Wellenfeld in Wechselwirkung steht und dass in Abwesenheit des Messgutes das Wellenfeld auf einen kleinen Bereich, den Fokalpunkt, fokussiert ist.

11. Verfahren nach Anspruch 7, dadurch gekennzeichnet, dass das Messgut mit einem linearen Wellenfeld in Wechselwirkung steht und dass in Abwesenheit des Messgutes das Wellenfeld auf einen kleinen Bereich, die Fokallinie, fokussiert ist.

12. Verfahren nach Anspruch 10 oder 11, dadurch gekennzeichnet, dass die Wellen-Intensitätsverteilung in der Fokalebene des fokussierten Wellenfeldes als Raumfrequenzspektrum gemessen wird.

13. Verfahren nach Anspruch 12, dadurch gekennzeichnet, dass das Raumfrequenzspektrum des durch das Messgut gestörten Wellenfeldes an vorgegebenen festen Messpunkten gemessen wird.

14. Verfahren nach Anspruch 12, dadurch gekennzeichnet, dass das Raumfrequenzspektrum durch Abtasten der Fokalebene gemessen wird.

15. Verfahren nach den vorangehenden Ansprüchen, dadurch gekennzeichnet, dass das Wellenfeld ein optisches ist.

16. Verfahren nach den Ansprüchen 1 - 14, dadurch gekennzeichnet, dass das Wellenfeld ein akustisches ist.

17. Verfahren nach Anspruch 16, dadurch gekennzeichnet, dass es ein Ultraschall-Wellenfeld ist.

18. Verfahren nach Anspruch 17, dadurch gekennzeichnet, dass ein Ultraschallsender mittels eines Ultraschall-Pulsgenerators ein gepulstes Ultraschallsignal abgibt und das vom Fremdgut reflektierte Signal von einem Ultraschallempfänger detektiert wird und dass aufgrund der Unterschiede in den beiden Signalen mit Hilfe des Korrelators eine Strukturanalyse des Messgutes durchgeführt wird.

19. Verfahren nach Anspruch 18, dadurch gekennzeichnet, dass auf der dem Ultraschallsender bezüglich des Messgutes gegenüberliegenden Seite ein weiterer Ultraschallempfänger liegt, der das durch das Messgut transmittierte Ultraschallpuls-Signal aufnimmt.

20. Verfahren nach Anspruch 19, dadurch gekennzeichnet, dass die Signalverarbeitung des reflektierten Ultraschallpulses in Funktion des transmittierten Ultraschallpuls-Signals geregelt wird.

21. Vorrichtung zur Erkennung von Fremdgut (5,6,7) in einem Fasergut- oder Messgutstrom (2) in der Textilindustrie, insbesondere Spinnerei, mit Transportmitteln (117,119, 120;130,131;131,135,132), welche den Messgutstrom (2) in Form eines Luft-Faser-Gemisches in einem Transportrohr (8) oder in Form eines Vlieses (118) bewegen, dadurch gekennzeichnet, dass eine den Messgutstrom (2) erfassende Messvorrichtung (10,30;50;50.1;60) vorgesehen ist, welche mit dem Messgut (1) mit einem vorgegebenen Wellenfeld in Wechselwirkung tritt, dessen Wellenlängen in der Grössenordnung der typischen Dimensionen des nachzuweisenden Fremdguts liegen, und welche mindestens einen Wellengenerator (11,12;31;32;51) für die Erzeugung der betreffenden Wellenlängen sowie eine Wellenempfangsvorrichtung (17,37) und eine Wellenaufbereitungsvorrichtung (18;38;57) aufweist.

22. Vorrichtung nach Anspruch 21, dadurch gekennzeichnet, dass die Wellenaufbereitungsvorrichtung (18; 38; 58) mit einer Display-Vorrichtung (19; 39) und/oder mit einer Abscheidevorrichtung (59; 110) für das Ausscheiden von Fremdgut aus dem Messgutstrom (2) verbunden ist.

23. Vorrichtung nach Anspruch 21, dadurch gekennzeichnet, dass die Messvorrichtung (10; 30; 50; 50.1; 60) zusätzlich mit einer Wellenoptik (13, 16; 33, 36; 62, 66) versehen ist, die beim Messgutstrom (2) angebracht ist.

24. Vorrichtung nach Anspruch 21, dadurch gekennzeichnet, dass die Messvorrichtung eine optische Messvorrichtung (10) ist.

25. Vorrichtung nach Anspruch 24, dadurch gekennzeichnet, dass die Messvorrichtung (10) ein Steuergerät (11) für eine Lichtquelle (12) (auch Emitter genannt) sowie einen Lichtdetektor (17) und eine Signalaufbereitungsanlage (18) umfasst.

26. Vorrichtung nach Anspruch 25, dadurch gekennzeichnet, dass die Messvorrichtung 10 zusätzlich eine Lichtemitteroptik (13) und eine Detektoroptik (16) umfasst.

27. Vorrichtung nach Anspruch 25, dadurch gekennzeichnet, dass die Lichtquelle eine Laserquelle ist.

28. Vorrichtung nach Anspruch 21, dadurch gekennzeichnet, dass die Messvorrichtung eine akustische Messvorrichtung (30; 50; 50.1; 60) ist.

29. Vorrichtung nach Anspruch 28, dadurch gekennzeichnet, dass die Messvorrichtung (30; 50; 50.1; 60) eine Ultraschall-Messvorrichtung ist.

30. Vorrichtung nach Anspruch 29, dadurch gekennzeichnet, dass die Ultraschallmessvorrichtung zusätzlich eine Ultraschalloptik des Empfängers unmittelbar nach dem Messgutstrom umfasst.

31. Vorrichtung nach Anspruch 30, dadurch gekennzeichnet, dass die Ultraschallmessvorrichtung zusätzlich noch eine Ultraschalloptik des Senders unmittelbar vor dem Messgutstrom umfasst.

32. Vorrichtung nach Anspruch 29, dadurch gekennzeichnet, dass die Ultraschallmessvorrichtung eine Ultraschall-Reflexions-Messvorrichtung (50) ist, welche einen die Zeitdifferenz und Stärkeunterschied zwischen einem Ultraschallpuls und einem Echopuls verarbeitenden Korrelator (58) umfasst und dieser Korrelator mit der Display-vorrichtung mit der Abscheidevorrichtung (59) verbunden ist.

33. Vorrichtung nach Anspruch 32, dadurch gekennzeichnet, dass der Korrelator ausserdem noch mit einer Display-Vorrichtung verbunden ist.

34. Vorrichtung nach einem der Ansprüche 21 - 23, dadurch gekennzeichnet, dass die Wellenmessvorrichtung ein Raumfrequenz-Analysator (60) ist, welcher mit einer Mehrzahl von Wellensendern (32) sowie einer entsprechenden Anzahl Wellenempfängern (37) und einer dazwischenliegenden fokussierenden Wellenoptik (66) versehen ist.

35. Vorrichtung nach einem der Ansprüche 21 - 23, dadurch gekennzeichnet, dass die Wellenmessvorrichtung eine Kombination von transmittierender und reflektierender Messvorrichtung (50.1) ist, welche einen Korrelator aufweist.

36. Vorrichtung nach einem der vorangehenden Ansprüche, dadurch gekennzeichnet, dass die Messvorrichtung mit einer Ausscheidevorrichtung (59) und/oder mit einer Displayvorrichtung (39) verbunden ist.

37. Vorrichtung nach einem der vorhergehenden Ansprüche, dadurch gekennzeichnet, dass die Ausscheidevorrichtung (59) eine Rohrweiche (104) in einen den Messgutstrom aufnehmenden pneumatischen Förderrohr ist, welche von der Messvorrichtung gesteuert ist.

38. Vorrichtung nach einem der vorangehenden Ansprüche, dadurch gekennzeichnet, dass die Ausscheidevorrichtung drei Klemmwalzenpaare umfasst, welche in Förderrichtung eines dazwischen geklemmten Faservlieses hintereinander angeordnet sind und, dass die Messvorrichtung zwischen dem ersten (117) und zweiten (119) Klemmwalzenpaar und die Ausscheidevorrichtung (122, 123, 124, 125) zwischen dem zweiten (119) und dritten (120) Klemmwalzenpaar vorgesehen ist, wobei die Ausscheidevorrichtung eine vorgegebene Anzahl Düsen umfasst, welche quer zur Förderrichtung des Faservlieses in einer Reihe angeordnet sind und, dass die Austrittsmündung dieser Blasdüsen (124) unmittelbar über dem Faservlies (118) angeordnet ist, sowie dass auf der den Blasdüsen gegenüberliegenden Seite des Faservlieses eine gleiche Anzahl Saugdüsen in derselben Reihe und derselben Art angeordnet sind, sowie dass die Intensität und der Zeitpunkt sowie die Auswahl der entsprechenden Blasdüse durch die Messvorrichtung gesteuert ist.

39. Vorrichtung nach Anspruch 38, dadurch gekennzeichnet, dass dem ersten und zweiten Klemmwalzenpaar je ein Förderband (130, 131) zugeordnet ist, welches je das Faservlies dem entsprechenden Klemmwalzenpaar (133, 135) zuführt und, dass die Messvorrichtung zwischen dem ersten Klemmwalzenpaar (133) und dem Förderband des zweiten Klemmwalzenpaares (135) angeordnet ist.

40. Vorrichtung zur Erkennung von Fremdgut in einem Fasergutstrom der Textilindustrie insbesondere Spinnerei gekennzeichnet durch Mittel zum Abgeben und Empfangen von Strahlung einer den Dimensionen der festzustellenden Teile angepassten Wellenlänge.

41. Vorrichtung nach einem der vorangehenden Ansprüche, dadurch gekennzeichnet, dass der Fasergutstrom im pneumatischen Transportsystem der Putzerei einer Spinnereianlage fliesst.

42. Vorrichtung zur Erkennung von Fremdgut in einem Faser- bzw. Flockenstrom in einer Spinnereianlage, gekennzeichnet, durch einen Strahlungssender/-empfänger, der auf Faser bzw. Flocken mit einem relativ weich strukturierten Bild und auf Fremdgut mit einem relativ scharf strukturierten Bild reagiert.

43. Verfahren nach Anspruch 1,
dadurch gekennzeichnet, dass der Wellenlängenbereich von etwa 0,1 mm bis 3,5 mm ist.

## Claims

1. A method for recognizing foreign material in a stream of fibre material (also called stream of measured material herein) in the textile industry, in form of an air-fibre mixture or in form of a fleece, whereby the first is conveyed in a pneumatical conveying tube and the second is conveyed freely or mechanically movably, characterized in
- that the stream of fibre material interacts with a predefined wave field whose wavelength is within the magnitude of the typical dimensions of the foreign material to be determined and which is formed by a respective wave generator which is in connection with a signal generator
and
- that the wave field of the wave receivers is altered by the dispersion of the waves owing to the interaction between measured material and the wave field, and
- that the information about the structure of the foreign material to be proven is obtained from the electric signals of the wave receivers.

2. A method as claimed in claim 1, characterized in that the wave field of the wave generator is focused by wave optics.

3. A method as claimed in claim 1, characterized in that the wave field is changed by wave dispersion and/or absorption.

4. A method as claimed in claim 1, characterized in that the signals are prepared.

5. A method as claimed in claim 1, characterized in that the wave field changed by the stream of fibre material is received by wave optics of wave converters after the influence of waves.

6. A method as claimed in claim 1, characterized in that a wave signal is issued continuously from at least one wave converter and that the wave signal influenced by the measured material is received by means of at least on further wave converter and that the wave signal reduced or dispersed by the measured material is measured.

7. A method as claimed in claim 6, characterized in that a plane wave field which is expanded in comparison with the transversal dimensions of the stream of measured material interacts with the measured material.

8. A method as claimed in claim 6, characterized in that a wave field which is linear and expanded in comparison with the transversal dimensions of the stream of measured material interacts with the measured material.

9. A method as claimed in claim 6, characterized in that the measured material interacts with a convergent wave field whose focal point is situated outside of the stream of measured material.

10. A method as claimed in claim 7, characterized in that the measured material interacts with a plane wave field and that in the event of the absence of the measured material the wave field is focused in a small area, i.e. the focal point.

11. A method as claimed in claim 7, characterized in that the measured material interacts with a linear wave field and that in the absence of the measured material the wave field is focused in a small area, i.e. the focal line.

12. A method as claimed in claim 10 or 11, characterized in that wave intensity distribution in the focal plane of the focused wave field is measured as a space frequency spectrum.

13. A method as claimed in claim 12, characterized in that the space frequency spectrum of the wave field distorted by the measured material is measured at predefined fixed measuring points.

14. A method as claimed in claim 12, characterized in that the space frequency spectrum is measured by scanning the focal plane.

15. A method as claimed in the previous claims, characterized in that the wave field is optical.

16. A method as claimed in claims 1 to 14, characterized in that the wave field is acoustic.

17. A method as claimed in claim 16, characterized in that it is an ultrasonic wave field.

18. A method as claimed in claim 17, characterized in that an ultrasonic transmitter issues a pulsed ultrasonic signal by means of an ultrasonic pulse generator and that the signal reflected by the foreign material is detected by an ultrasonic receiver and that due to the difference in the two signals a structural analysis of the measured material is made with the help of the correlator.

19. A method as claimed in claim 18, characterized in that on the side which is opposite of the ultrasonic transmitter with respect to the measured material there is a further ultrasonic receiver which receives the ultrasonic pulse signal transmitted by the measured material.

20. A method as claimed in claim 19, characterized in that the signal processing of the reflected ultrasonic pulse is controlled as a function of the transmitted ultrasonic pulse signal.

21. An apparatus for recognizing foreign material (5, 6, 7) in a stream of fibres or measured material (2) in the textile industry, in particular a spinning mill, with conveying means (117, 119, 120; 130, 131; 131, 135, 132) which move the stream of measured material (2) in form of an air-fibre mixture in a conveying tube (8) or in form of a fleece (118), characterized in that a measuring apparatus (10, 30; 50; 50.1; 60) detecting the stream of measured material (2) is provided which interacts with the measured material (1) with a predefined wave field whose wavelengths are within the magnitude of the typical dimensions of the foreign material to be proven and which comprises at least one wave generator (11, 12; 31; 32; 51) for the generation of the respective wavelengths as well as a wave receiving apparatus (17, 37) and a wave preparation apparatus (18, 38; 57).

22. An apparatus as claimed in claim 21, characterized in that the wave preparation apparatus (18; 38; 58) is connected to a display apparatus (19; 39) and/or a separating apparatus (59; 110) for separating foreign material from the stream of measured material (2).

23. An apparatus as claimed in claim 21, characterized in that the measuring apparatus (10; 30; 50; 50.1; 60) is additionally provided with wave optics (13, 16; 33, 36; 62, 66) which is attached near the stream of measured material.

24. An apparatus as claimed in claim 21, characterized in that the measuring apparatus is an optical measuring apparatus (10).

25. An apparatus as claimed in claim 24, characterized in that the measuring apparatus (10) comprises a control device (11) for a light source (12) (also called an emitter herein) as well as a light detector (17) and a signal processing system (18).

26. An apparatus as claimed in claim 25, characterized in that the measuring apparatus (10) comprises in addition light emitter optics (13) and detector optics (16).

27. An apparatus as claimed in claim 25, characterized in that the light source is a laser source.

28. An apparatus as claimed in claim 21, characterized in that the measuring apparatus is an acoustic measuring apparatus (30; 50; 50.1; 60).

29. An apparatus as claimed in claim 28, characterized in that the measuring apparatus (30; 50; 50.1; 60) is an ultrasonic measuring apparatus.

30. An apparatus as claimed in claim 29, characterized in that the ultrasonic measuring apparatus comprises ultrasonic optics of the receiver instantly after the stream of measured material.

31. An apparatus as claimed in claim 30, characterized in that the ultrasonic measuring apparatus comprises in addition ultrasonic optics of the transmitter directly in front of the stream of measured material.

32. An apparatus as claimed in claim 29, characterized in that the ultrasonic measuring apparatus is an ultrasonic reflection measuring apparatus (50) which comprises a correlator (58) processing a time difference and difference in strength between an ultrasonic pulse and an echo pulse and that said correlator is connected with the display apparatus with the separating apparatus (59).

33. An apparatus as claimed in claim 32, characterized in that the correlator is also connected with a display apparatus.

34. An apparatus as claimed in one of the claims 21 to 23, characterized in that the wave measuring apparatus is a space frequency analyzer (60) which is provided with a plurality of wave transmitters (32) as well as a respective number of wave receivers (37) and interposed focusing wave optics (66).

35. An apparatus as claimed in one of the claims 21 to 23, characterized in that the wave measuring apparatus is a combination of a transmitting and reflecting measuring apparatus (50.1) which comprises a correlator.

36. An apparatus as claimed in one of the previous claims, characterized in that the measuring apparatus is connected to a separating apparatus (59) and/or to a display apparatus (39).

37. An apparatus as claimed in one of the previous claims, characterized in that the separating apparatus (59) is a tube branch (104) in a pneumatic conveying tube receiving the stream of measured material, which tube is controlled by the measuring apparatus.

38. An apparatus as claimed in one of the previous claims, characterized in that the separating apparatus comprises three pairs of nip rollers which are arranged behind one another in the conveying direction of a fibre fleece nipped between said rollers and that the measuring apparatus is provided between the first (117) and the second (119) pair of nip rollers and the separating apparatus (122, 123, 124, 125) is provided between the second (119) and the third (120) pair of nip rollers, whereby the separating apparatus comprises a predefined number of nozzles which are arranged in a row transversal to the conveying direction of the fibre fleece and that the discharge openings of said blower nozzles (124) are arranged directly above the fibre fleece (118) and that on the side of the fibre fleece opposite of the blower nozzles there are arranged a similar number of suction nozzles in the same row and in the same manner and that the selection of the respective blower nozzle is controlled by the measuring apparatus.

39. An apparatus as claimed in claim 38, characterized in that a conveyor belt (130/131) is allocated both to the first and the second pair of nip rollers which each supplies the fibre fleece to the respective pair of nip rollers (133, 135) and that the measuring apparatus is arranged between the first pair of nip rollers (133) and the conveyor belt of the second pair of nip rollers (135).

40. An apparatus for recognizing foreign material in a stream of fibre material in the textile industry, in particular a spinning mill, characterized by means for issuing and receiving radiation of wavelengths adapted to the dimensions of the particles to be determined.

41. An apparatus as claimed in one of the previous claims, characterized in that the stream of fibre material flows in the pneumatic conveying system of the blow room of a spinning plant.

42. An apparatus for recognizing foreign material in a stream of fibres or flocks in a spinning plant, characterized by a radiation transmitter/receiver which reacts to fibres or flocks with a relatively softly structured image and to foreign material with a relatively sharply structured image.

43. An method as claimed in claim 1, characterized in that the range of the wave field is from approximately 0,1 mm to 3,5 mm.

## Revendications

1. Procédé servant à reconnaître la présence d'une matière étrangère dans un courant de matière fibreuse (appelé également courant de matière a mesurer) dans l'industrie textile, sous forme d'un mélange air-fibres ou sous forme d'un voile, procédé dans lequel le premier est transporté dans un tuyau de transport pneumatique, et le deuxième est transporté librement ou pouvant être mû mécaniquement,
caractérisé par le fait
- que le courant de matière fibreuse se tient en dialogue avec un champ d'ondes prédéterminé, dont les longueurs d'ondes se situent dans l'ordre de grandeur des dimensions types de la matière étrangère à détecter et qui est formé par un générateur d'ondes correspondant qui se trouve en liaison avec un générateur de signaux, et
- que le champ d'ondes, qui est changé à cause d'une dispersion d'ondes provoquée par le dialogue entre la matière à mesurer et le champ d'ondes, est réceptionné par des récepteurs d'ondes, et
- que l'information des signaux électriques concernant la structure de la matière étrangère à détecter est réceptionnée par les récepteurs d'ondes.

2. Procédé selon revendication 1,
caractérisé par le fait que
le champ d'ondes du générateur d'ondes est mis en faisceau par une optique ondulatoire.

3. Procédé selon revendication 1,
caractérisé par le fait que
le champ d'ondes est changé par une disperson d'ondes et/ou par absorption.

4. Procédé selon revendication 1,
caractérisé par le fait que
les signaux sont traités.

5. Procédé selon revendication 1,
caractérisé par le fait que
le champ d'ondes, changé par le courant de matière fibreuse, après une influence d'ondes, est réceptionné par une optique ondulatoire des convertisseurs d'ondes.

6. Procédé selon revendication 1,
caractérisé par le fait
qu'un signal ondulé est émis continuellement par au moins un convertisseur d'ondes, et que le signal ondulé, influencé par la matière à mesurer, est reçu au moyen d'au moins un autre convertisseur d'ondes, et que le signal ondulé, affaibli ou dispersé par la matière a mesurer, est mesuré.

7. Procédé selon revendication 6,
caractérisé par le fait
qu'un champ d'ondes aplani, étendu en comparaison avec les dimensions transversales du courant de matière a mesurer, se tient en dialogue avec la matière a mesurer.

8. Procédé selon revendication 6,
caractérisé par le fait
qu'un champ d'ondes linéaire, étendu en comparaison avec les dimensions transversales du courant de matière à mesurer, se tient en dialogue avec la matière à mesurer.

9. Procédé selon revendication 6,
caractérisé par le fait que
la matière à mesurer se tient en dialogue avec un champ d'ondes convergent, dont le point focal se situe en dehors du courant de matière à mesurer.

10. Procédé selon revendication 7,
caractérisé par le fait que
la matière à mesurer se tient en dialogue avec un champ d'ondes aplani, et que, en absence de matière à mesurer, le champ d'ondes est focalisé sur une petite zone, le point focal.

11. Procédé selon revendication 7,
caractérisé par le fait que
la matière à mesurer se tient en dialogue avec un champ d'ondes linéaire, et que, en absence de matière à mesurer, le champ d'ondes est focalisé sur une petite zone, la ligne focale.

12. Procédé selon revendication 10 ou 11,
caractérisé par le fait que
la répartition des intensités d'ondes est mesurée dans le plan focal du champ d'ondes focalisé, comme spectre de fréquences spaciales.

13. Procédé selon revendication 12,
caractérisé par le fait que
le spectre de fréquences spaciales du champ d'ondes perturbé par la matière à mesurer est mesuré à des points de mesure fixes prédéterminés.

14. Procédé selon revendication 12,
caractérisé par le fait que
le spectre de fréquences spaciales est mesuré par balayage du plan focal.

15. Procédé selon les revendications précédentes,
caractérisé par le fait que
le champ d'ondes est optique.

16. Procédé selon les revendications 1 à 14,
caractérisé par le fait que
le champ d'ondes est acoustique.

17. Procédé selon revendication 16,
caractérisé par le fait que
le champ d'ondes est un champ d'ondes ultrason.

18. Procédé selon revendication 17,
caractérisé par le fait
qu'un émetteur ultrason émet un signal ultrason pulsé au moyen d'un générateur d'impulsions ultrason, et que le signal réfléchi par la matière étrangère est détecté par un récepteur ultrason, qu'une analyse de structure de la matière à mesurer est effectuée en fonction des différences dans les deux signaux, à l'aide du corrélateur.

19. Procédé selon revendication 18,
caractérisé par le fait que,
sur le côté opposé a l'émetteur ultrason relatif à la matière a mesurer, est situé un autre récepteur ultrason qui réceptionne le signal d'impulsions ultrason transmis par la matière à mesurer.

20. Procédé selon revendication 19,
caractérisé par le fait que
le traitement du signal d'impulsions ultrason réfléchi est réglé en fonction du signal d'impulsions ultrason transmise.

21. Dispositif servant à reconnaître la présence de matière étrangère (5, 6, 7) dans un courant de matière fibreuse ou de matière à mesurer (2) dans l'industrie textile, particulièrement en filature, ayant des moyens de transport (117, 119, 120; 130, 131; 131, 135, 132) qui mettent en mouvement le courant de matière à mesurer (2) sous forme d'un mélange air-fibres dans un tuyau de transport (8) ou sous forme d'un voile (118),
caractérisé par le fait
qu'un dispositif de mesure (10, 30; 50; 50.1; 60) est prévu, détectant le courant de matière a mesurer (2), qui, avec la matière à mesurer (1), entre en dialogue avec un champ d'ondes prédéterminé dont les longueurs d'ondes se situent dans l'ordre de grandeur des dimensions types de la matière étrangère à détecter, et qui possède au moins un générateur d'ondes (11, 12; 31; 32; 51) pour produire les longueurs d'ondes correspondantes, ainsi qu'un dispositif récepteur d'ondes (17, 37) et un dispositif de traitement d'ondes (18; 38; 57).

22. Dispositif selon revendication 21,
caractérisé par le fait que
le dispositif de traitement d'ondes (18; 38; 58) est relié avec un dispositif Display (19; 39) et/ou un dispositif séparateur (59; 110) servant à séparer la matière étrangère du courant de matière à mesurer (2).

23. Dispositif selon revendication 21,
caractérisé par le fait que
le dispositif de mesure (10; 30; 50; 50.1; 60) est pourvu d'une optique ondulatoire supplémentaire (13, 16; 33, 36; 62, 66) qui est disposée auprès du courant de matière à mesurer (2).

24. Dispositif selon revendication 21,
caractérisé par le fait que
le dispositif de mesure est un dispositif de mesure optique (10).

25. Dispositif selon revendication 24,
caractérisé par le fait que
le dispositif de mesure (10) comprend un appareil de commande (11) pour une source de lumière (12) (appelée également émetteur) ainsi qu'un détecteur de lumière (17) et une installation de traitement de signaux (18).

26. Dispositif selon revendication 25,
caractérisé par le fait que
le dispositif de mesure (10) comprend en plus une optique émettant de la lumière (13) et une optique détectant la lumière (16).

27. Dispositif selon revendication 25,
caractérisé par le fait que
la source de lumière est une source laser.

28. Dispositif selon revendication 21,
caractérisé par le fait que
le dispositif de mesure est un dispositif de mesure acoustique (30; 50; 50.1; 60).

29. Dispositif selon revendication 28,
caractérisé par le fait que
le dispositif de mesure (30; 50; 50.1; 60) est un dispositif de mesure ultrason.

30. Dispositif selon revendication 29,
caractérisé par le fait que
le dispositif de mesure ultrason comprend en plus une optique ultrason du récepteur, placée immédiatement après le courant de matière à mesurer.

31. Dispositif selon revendication 30,
caractérisé par le fait que
le dispositif de mesure ultrason comprend encore en plus une optique ultrason de l'émetteur, placée immédiatement avant le courant de matière à mesurer.

32. Dispositif selon revendication 29,
caractérisé par le fait que
le dispositif de mesure ultrason est un dispositif de mesure ultrason réfléchissant (50) qui comprend un corrélateur (58) traitant la différence de temps et la différence de puissance entre une impulsion ultrason et une impulsion d'écho, et ce corrélateur est relié avec le dispositif Display et avec le dispositif séparateur (59).

33. Dispositif selon revendication 32,
caractérisé par le fait que
le corrélateur est, en outre, encore relié avec un dispositif Display.

34. Dispositif selon l'une des revendications 21 à 23,
caractérisé par le fait que
le dispositif de mesure d'ondes est un analyseur de fréquences spaciales (60), lequel est pourvu d'une pluralité d'émetteurs d'ondes (32) ainsi que d'un nombre correspondant de récepteurs d'ondes (37) et d'une optique ondulatoire (66) focalisante, située entre ceux-ci.

35. Dispositif selon l'une des revendications 21 à 23,
caractérisé par le fait que
le dispositif de mesure d'ondes est une combinaison de dispositifs de mesure transmetteur et réflecteur (50.1), laquelle possède un corrélateur.

36. Dispositif selon l'une des revendications précédentes,
caractérisé par le fait que
le dispositif de mesure est relié avec un dispositif séparateur (59) et/ou avec un dispositif Display (39).

37. Dispositif selon l'une des revendications précédentes,
caractérisé par le fait que
le dispositif séparateur (59) est un aiguillage de tuyaux (104) situé dans un tuyau de transport pneumatique recevant le courant de matière à mesurer, aiguillage qui est commandé par le dispositif de mesure.

38. Dispositif selon l'une des revendications précédentes,
caractérisé par le fait que
le dispositif séparateur comprend trois paires de rouleaux de pincement disposées l'une derrière l'autre dans le sens de transport d'un voile de fibres pincé entre celles-ci, et que le dispositif de mesure est prévu entre la première paire de rouleaux de pincement (117) et la deuxième (119), et le dispositif séparateur (122, 123, 124, 125) est prévu entre la deuxième paire de rouleaux de pincement (119) et la troisième (120), et où le dispositif séparateur comprend un nombre prédéterminé de buses qui sont disposées en une rangée, perpendiculairement au sens de transport du voile de fibres, que l'embouchure de sortie de ces buses de soufflage (124) est disposée immédiatement au-dessus du voile de fibres (118), et qu'un nombre égal de buses d'aspiration est disposé du côté du voile de fibres opposé aux buses de soufflage, dans le même alignement et dans le même ordre, de même que l'intensité et le temps de soufflage, ainsi que le choix de la buse de soufflage correspondante, sont commandés par le dispositif de mesure.

39. Dispositif selon revendication 38,
caractérisé par le fait
qu'une bande transporteuse (130, 131) est adjointe à chacune des première et deuxième paires de rouleaux de pincement, qui amène chacune le voile de fibres vers la paire de rouleaux de pincement correspondante (133, 135), et que le dispositif de mesure est disposé entre la première paire de rouleaux de pincement (133) et la bande transporteuse de la deuxième paire de rouleaux de pincement (135).

40. Dispositif servant a reconnaître la présence d'une matière étrangère dans un courant de matière de fibres de l'industrie textile, particulièrement en filature,
caractérisé par
des moyens utilisés pour émettre et recevoir le rayonnement d'une longueur d'ondes adaptée aux dimensions des parties à détecter.

41. Dispositif selon l'une des revendications précédentes,
caractérisé par le fait que
le courant de matière fibreuse s'écoule dans le système de transport pneumatique de battage-nettoyage d'une installation de filature.

42. Dispositif servant à reconnaître la présence d'une matière étrangère dans un courant de fibres respectivement de flocons d'une installation de filature,
caractérisé par
un émetteur/récepteur de rayons qui réagit à des fibres respectivement des flocons ayant une structure relativement tendre, et à une matière étrangère qui possède une structure relativement dure.

43. Procédé selon revendication 1,
caractérisé par le fait que
la zone de longueurs d'ondes se situe depuis environ 0,1 mm jusqu'à 3,5 mm.
